Europäisches Patentamt

European Patent Office

Office européen des brevets

(19)

(11) Publication number: **0 059 113**
**B1**

(12) **EUROPEAN PATENT SPECIFICATION**

(45) Date of publication of patent specification: **15.10.86**

(21) Application number: **82300964.2**

(22) Date of filing: **25.02.82**

(51) Int. Cl.⁴: **C 12 N 1/20,** C 12 N 1/00, C 12 N 1/38

(54) Improved bacterial growth medium and method of growing bacteria.

(30) Priority: **25.02.81 US 237883**

(43) Date of publication of application:
**01.09.82 Bulletin 82/35**

(45) Publication of the grant of the patent:
**15.10.86 Bulletin 86/42**

(84) Designated Contracting States:
**AT BE CH DE FR GB IT LI LU NL SE**

(56) References cited:
EP-A-0 022 341
GB-A- 463 720
GB-A-1 125 997
US-A-3 354 049

(73) Proprietor: **STATE OF OREGON, by and through the Oregon State Board of Higher Education on behalf of Oregon State University**
**Corvallis, Oregon 97330 (US)**

(72) Inventor: **Sandine, William E.**
**2690 N.W. Royal Oaks Drive**
**Corvallis Oregon 97331 (US)**
Inventor: **Ayres, James W.**
**1173 Charlemegne Place**
**Corvallis, Oregon 97330 (US)**

(74) Representative: **Brown, John David et al**
**FORRESTER & BOEHMERT Widenmayerstrasse 4/I**
**D-8000 München 22 (DE)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

## Description

### Background of the invention

#### 1. Field of the invention

The present invention relates to an improved method and compositions for growing acid producing bacteria wherein an essentially water insoluble alkaline earth metal salt or base is generated *in situ* in the aqueous growth medium. In particular, the present invention relates to the use of a powdered growth medium composition containing an alkaline earth metal cation and an anion which reacts to form the insoluble salt or base when the powder is added to an aqueous solution.

#### 2. Prior art and related patent application

In EP—A—0 022 341, we described prior art relating to neutralization in growing acid producing bacteria. This earlier application particularly describes an improvement in the use of a water insoluble base or salt as a neutralizing agent for the acid generated by the bacteria. In particular, EP—A—0 022 341 discloses a method of growing acid producing bacteria in an aqueous growth medium including bacterial neutrients, in which an essentially water insoluble salt, base or mixture thereof including an alkaline earth metal cation is added to the aqueous growth medium, which essentially water insoluble salt, base or mixture thereof is acid neutralizing in the medium, wherein the resulting medium includes an amount of the insoluble salt, base or mixture thereof in the medium sufficient to neutralize at least some of the acid generated by the acid producing bacteria and wherein the resulting medium is non-toxic to the bacteria. This method works extremely well; however, various considerations, particularly cost and availability of chemicals as discussed hereinafter, dictated finding a method wherein the insoluble neutralizing agent was prepared in a different manner. Also, the insoluble salts and bases may be granular rather than powdered, or require hammer milling to prevent mixing and dispersion problems both before and during use of the compositions, which may limit their effectiveness.

The reaction of compounds in water to produce soluble or insoluble salts is well known in the general chemical literature. However, the inventors are not aware of any prior art which deals with the *in situ* generation of an insoluble neutralizing agent in a bacterial growth media.

Also, the inventors are not aware that it has ever previously been proposed to neutralize acid in a bacterial growth medium by the addition of an alkaline earth metal cation which is initially in soluble form. It is known that such cations can precipitate bacterial nutrients such as lactose and sucrose from solution (see, for example, Journal of Food Science—Volume 45 (1980), pp 362 et seq.).

### Objects

It is therefore an object of the present invention to provide a method wherein an insoluble alkaline earth metal salt or base as a neutralizing agent is generated *in situ* in an aqueous growth medium when selected compounds are added to the aqueous growth medium, wherein the resulting medium is very effective for growing acid producing bacteria. It is further an object of the present invention to provide a method to generate essentially water insoluble neutralizing agents *in situ* by chemical reaction of appropriate compounds in the aqueous growth medium, so as to maintain a pH which minimizes the adverse effects of acid produced during bacterial growth and therefore maximises the number and fermentation activity of the cells. It is further an object of the present invention to provide a method and compositions which generate a well dispersed insoluble salt or base as a neutralizing agent in the aqueous growth medium. Further still, it is an object of the present invention to provide a method and compositions which are very economical and use readily available chemicals. These and other objects will become increasingly apparent by reference to the following description.

### General description

The present invention relates to an improvement in the method for growing acid producing bacteria in an aqueous growth medium including bacterial nutrients which comprises reacting in the aqueous growth medium a water soluble alkaline earth metal cation in a compound A with an anion in a compound B which reacts with the alkaline earth metal cation to form an essentially water insoluble salt, base or mixture thereof, including the alkaline earth metal cation and the anion, which is acid neutralizing in the medium, wherein the resulting medium is adapted to provide an amount of the insoluble salt or base or mixture thereof in the aqueous medium sufficient to neutralize at least some of the acid generated by the acid producing bacteria and wherein the resulting medium is non-toxic to the bacteria.

The present invention further relates to an improved bacterial growth medium composition adapted for growth of bacteria in an aqueous growth medium which comprises a powdered growth medium adapted to be admixed with water including a water soluble alkaline earth metal cation in a compound A admixed with an anion in compound B in an amount sufficient to react in water to form an essentially water insoluble salt, base or mixture thereof, including the alkaline earth metal cation and the anion, wherein the powdered growth medium is adapted to provide an amount of the insoluble salt, base or mixture thereof in the aqueous medium sufficient to neutralize at least some of the acid generated by the acid producing bacteria and wherein the growth medium when admixed with water has an initial pH between about 4 and 8.5 and is non-toxic to the bacteria

EP—A—0 022 341 relates to a method and to compositions for growing acid producing bacteria by using an essentially water insoluble or temporarily water insolubilised and thus initially solid form of an acid neutralising agent in the growth medium to provide a controlled reaction with acid produced by the bacteria without substantially raising the pH of the growth medium. The specific improvement of EP—A—0 022 341 involves the addition of essentially insoluble magnesium compounds such as trimagnesium phosphate, or other magnesium phosphate salts which are insoluble under neutral and near neutral conditions (pH 6.5—7.5) with acid generating bacterial cultures used as starters in the fermentation industry. As the bacteria grow and acid is produced, the insoluble neutralizing agent is solubilised thereby immediately neutralizing the acids as they are produced. Because of the insoluble nature of the neutralizing agent it has essentially no influence on the soluble composition of the nutrient growth media and sufficient quantities may be added to neutralize all the acid produced during subsequent growth depending on the availability of growth factors on the medium and the nature of the bacteria. Therefore, the bacteria can grow to higher populations and do no suffer from significant acid injury during growth as would occur without the insoluble neutralizing agent. Consequently, bacteria generated in such media are poised to transform a substrate such as milk at a faster rate and with a minimum lag compared to bacteria produced where the acid is not neutralized.

Trimagnesium phosphate is available commercially on the market from a limited number of suppliers. Industrial processes which depend on extremely limited sources of essential ingredients are commercially untenable. Magnesium ammonium phosphate also is generally unavailable in bulk quantities for industrial use.

The present invention is an improvement over EP—A—0 022 341 the improvement occurs by chemically generating a desired insoluble neutralizing agent by chemical reaction when the ingredients are mixed in water or milk or other suitable liquids to provide a growth medium. Preferably the ingredients are in the form of powders for ease of dispersion in aqueous solution. There is a substantial economic advantage from using the present invention. Inhalation health hazards are reduced in one of the improved methods (Example 1 hereinafter) since dry blending of fine powders of the compounds of magnesium hydroxide or trimagnesium phosphate is not necessary. The importance of this improvement in working conditions is significant.

The present invention thus provides a method for generating an essentially water insoluble, non-toxic neutralizing agent by chemical reaction in a bacterial growth medium. This in situ generation of the insoluble neutralizing agent occurs after addition of water or milk or other suitable liquid which forms the desired insoluble neutralizing agent. The resulting insoluble neutralizing agent is a base or salt or mixture thereof and is adapted to provide a controlled reaction with the acid produced by the bacteria without substantially raising the pH of the growth medium. The neutralizing agent is generated in the growth medium prior to the growth of the bacteria and is present in an amount sufficient to maintain the pH at a level beneficial for bacterial growth.

Liquid bulk starter compositions for growing bacteria in media wherein the insoluble neutralizing agent has been generated in situ can be provided by the present invention. Further, bacterial composition with enhanced storability and viability because of the essentially insoluble neutralizing agent are produced. Both of these are described in the parent application.

The physical characteristics of the in situ generated insoluble neutralizing agents are in some instances different from neutralizing agents which are not generated in situ. In particular, the physical properties of the powdered product after hydration are typically such that a minimum of settling or suspending materials occurs during fermentation because of the fine dispersion of the insoluble salt (see Example 1 below). Further in some cases the invention allows use of chemicals which are readily and uniformly blended in commonly, commercially used tumbler bins, whereas neutralizing agents which are not generated in situ are now well adapted for uniform blending in tumbler bins because of density and particle size character. In some instances the invention allows production of powders by spray drying water soluble chemicals which are low in equipment abrasion when compared to spray drying of water insoluble neutralizing agents in suspension which was necessary prior to the invention. The method of in situ generation and the resulting aqueous growth medium are particularly adapted to growing lactic acid producing bacteria which are then used in making food and beverage fermented products for animals and humans.

The principal alkaline earth metal cations of compound A are magnesium and calcium with the former being preferred as described in EP—A—0 002 341. Other alkaline earth metal cations which can be used, particularly in non-food settings, are barium and strontium so long as the resulting salts of bases are non-toxic to the bacteria in the aqueous growth medium.

Compound B preferably includes a phosphate anion since these form insoluble salts with the alkaline earth metal cations and are very effective neutralizing agents. Other compounds B can include various anions such as hydroxide, carbonate and the like, providing the anion reaction product with the alkaline earth metals is essentially insoluble in water.

In general, the reaction of compound A and compound B will involve essentially stoichiometric molar amounts of each although an excess of one or the other can be tolerated depending upon the particular acid producing bacterium that is being grown so long as the reaction products in water are non-toxic to the bacteria. In general, the powdered growth medium compositions contain between about 5 and 30 weight percent of each of the compounds A and B. It will be appreciated that compounds A and/or B can be toxic to the bacteria at the levels used in the powdered growth medium but that once they react to form the insoluble compounds in the aqueous solution for growth of the bacteria that is not a problem. Preferably the compounds A and B are substantially water soluble.

In general, the pH of the growth medium with the insoluble salt or base or mixture thereof is between about 4 and 8.5 in water. Higher or lower pH levels can be used provided the acid producing bacteria are tolerant to the basicity or acidity. The insoluble compounds which are formed *in situ* in the aqueous growth solution can be alkaline earth metal hydroxides, phosphates, carbonates, citrates and the like. Preferred are magnesium phosphate tribasic, magnesium phosphate dibasic, magnesium orthophosphate, magnesium orthophosphate monohydrogen, magnesium pyrophosphate, calcium phosphate tribasic, calcium phosphate dibasic, calcium phosphate monobasic, calcium carbonate, magnesium carbonate, magnesium hydroxide, magnesium ammonium phosphate, and the like as insoluble compounds. The magnesium phosphates, including magnesium ammonium phosphate, are particularly preferred since these compounds tend to inhibit phage in an aqueous growth medium involving lactic acid producing bacteria which are sensitive to phage. This class of bacteria includes those which are used for producing products like buttermilk, yogurt, cottage cheese and the like.

The compounds A and B are preferably admixed with the other ingredients conventionally used in growth media in dry powdered forms so that the product is a powder. In general, the powdered growth media should be contained in relatively moisture secure packages to prevent premature reaction of the compounds A and B because of atmospheric moisture or accidental contact with water. Various well known moisture proof packs are suitable for this purpose.

The other nutrients used in liquid or powdered growth medium are well known and include an assimilable carbohydrate, a nitrogen source and usually essential minerals where the object is to increase the number of the bacteria. The carbohydrates are usually in the form of simple sugars such as lactose or glucose which are directly metabolized by the bacteria to produce the acids. The nitrogen source preferably includes various forms of yeast such as yeast extract or other sources of assimilable amino acids such as tryptone, casein, phytone, peptone and beef extract. The essential minerals vary from bacteria to bacteria but generally include trace amounts of transition metal salts such as manganese and magnesium salts. In the present invention, the insoluble magnesium salts can function to provide part of the magnesium requirement of the bacteria upon reaction with the acid. Many variations in growth media are described in the prior art and are well known to those skilled in the art.

The phrase "essentially water insoluble" means an insoluble compound as a base or salt which functions as an acid neutralizing agent and which has a solubility in water (having a neutral pH without the agent) at 25°C of less than about 10 grams per liter. Based upon the definition of the United States Pharmacompea IXI, 1975, compounds are "slightly soluble" at 1.0 grams to 10.0 grams per liter; very "slightly soluble" at 0.1 gram per liter to 1.0 gram per liter and "practically insoluble" or "insoluble" at less than 0.1 gram per liter The phrases "essentially water insoluble" or "water insoluble" as used herein cover all of these solubilities.

Specific description

Examples of the present improvement are set forth hereinafter and it is intended that they be only illustrative. Cultures used in some of the examples are deposited in the collection maintained in the Department of Microbiology, Oregon State University and are freely available without cost to the public.

Example 1

EP—A—0 022 341, clearly shows the advantage of using the magnesium phosphates in a growth media for growing acid producing bacteria to be used in fermenting foods. However, the magnesium phosphates are expensive and of limited availability commercially. In addition, production of these agents requires expensive equipment not always readily available and further the magnesium phosphates are extremely abrasive compounds and require special handling to avoid excessive equipment wear and damage if these compounds are to be prepared as dry powders for blending with commercial powdered bacterial growth media. The following example of the invention shows the *in situ* generation of the desired magnesium phosphate in a manner which avoids all the difficulties listed.

A growth medium was prepared by combining 322 kg (710 pounds) of a dry powdered material containing magnesium citrate (compound A), 423 kg (933 pounds) of dibasic ammonium phosphate (compound B), 82 kg (181 pounds) of yeast extract and 578 kg (1,275 pounds) of whey powder. The dry powder material containing magnesium citrate was prepared by adding 264.5 kg (583 pounds) of anhydrous citric acid to 757 l (200 gallons) of water followed by 324.5 kg (715 pounds) of a magnesium hydroxide aqueous slurry containing 58.3% solids, with stirring for 30—60 minutes until the reaction was essentially complete and then by spray drying.

The entire combination of dry powder was then tumble mixed in a bin and later used to grow acid producing bacteria for use in food fermentation.

The insoluble salt is essentially magnesium ammonium phosphate.

In growing bacteria the powder was generally mixed in stirring water (22.5—34 kg/379 l) (50—75 pounds/100 gallons)) heated to about 85°C for 45 minutes, cooled to about 24°C and inoculated with 1% by weight of a lactic acid producing bacteria (about $1 \times 10^9$ cells per ml) and allowed to ferment for about 12—20 hours. The initial growth medium pH was generally about 6.5—7.1 and the final pH was about 4.9—5.4. When compared to bacteria produced using growth media reported in Example 10 of EP—A—0 022 341 the bacteria grown using *in situ* generation of the neutralizing agent described above were essentially equivalent in activity and cell numbers.

The advantages of the above example are numerous. These include: (a) expensive and limited source magnesium ammonium phosphate or other magnesium phosphates need not be used, (b) the ratio of compounds A & B can readily be varied to optimise initial pH, final pH and neutralizing capacity with more flexibility, (c) the soluble or mostly soluble magnesium citrate produced by combining magnesium hydroxide and citric acid can be spray dried with minimal equipment wear and damage, (d) the powders of the medium can be tumble bin blended to produce a uniform product, (e) the physical properties of the powdered product after hydration are such that a minimum of settling or suspending materials occurs during fermentation because of the fine dispersion of the insoluble salt, (f) the heat produced by *in situ* generation of the neutralizing agents not only does not adversely affect the medium, but can contribute somewhat to energy savings in heating an aqueous medium mixture for bacterial growth, and (g) the heat evolved on addition of magnesium hydroxide to the citric acid solution is substantial and makes a definite contribution to the energy savings by chemically preheating the reaction mixture as is needed prior to spray drying. This is a preferred but an optional step.

Example 2

A powdered blend mixture was prepared containing about 17.7% trisodium citrate dihydrate and 8.2% magnesium hydroxide (which can react in water to form a magnesium citrate which acts in part as compound A), along with 21.2% ammonium phosphate monobasic and 5.9% ammonium phosphate dibasic (which can act as compound B), 6.0% yeast extract, and 41% whey by mixing the ingredients in a tumbler bin to produce 1,361 kg (3,000 pounds) of product. After preparation, the powder was added to stirred water (34 kg/379 l (75 pounds/100 gallons)), heated to 85°C for 45 minutes and allowed to cool to about 24°C prior to inoculation (1%) by weight with a lactic acid producing streptococus starter culture. This medium allows *in situ* formation of the insoluble salt magnesium ammonium phosphate through reaction after the medium is hydrated. Repeated field trials were conducted with the bacteria grown in this medium and they grew in large numbers and were highly active. It should be noted that the exact chemical reaction sequence and specific chemical composition of the neutralizing agent(s) may be complex.

Physical-chemical characteristics of the neutralizing agent generated in conjunction with other medium ingredients were somewhat different than expected. In a 1,136—1,893 l (300—500 gallon) starter tank, it was routine to find a dense cement-like mass on the bottom of the tank at the end of the 12—20 hour fermentation period. The mass sometimes had to be chipped out of the tanks with a hammer. This problem was not nearly so prevalent with the medium described in Example 1, even though very similar neutralizing agents were being regenerated *in situ*. Extremely vigorous agitation could prevent some of the hard cake formation, but the insoluble residue was still in excess by comparison to Example 1. The step-wise process involved in generating the neutralizing agent in Example 1 is thus preferred.

The trisodium citrate available citrate content of commercial product was extremely variable ranging from 5.8 to 24.5% for a four-fold variation rather than being extremely close to the desired 17.7%. Varying blending times of the dry powders for 10, 20 or 30 minutes did change the result. Thus, variation in final formulation due to variation associated with the ingredients was responsible for variable behaviour of the powdered growth medium in the field. This is in contrast to what was observed with the medium of Example 1. In production of the medium by Example 1, there was only a 1.4% citrate variation on a production basis and thus the ingredients were adapted to standard tumbler bin blending.

It should be noted that workmen complained when working with powdered magnesium hydroxide of nosebleeds and eye irritation due to powder aerosolization. There was less problem with Example 1 where the magnesium hydroxide is used as the slurry and now a powder. Also, production of magnesium hydroxide by spray drying the slurry is quite expensive as the abrasive powder wears out the spray nozzle. The magnesium citrate composition produced in Example 1 is less abrasive and the equipment lasts longer.

Example 3

A growth medium was prepared by combining 8.3% magnesium hydroxide, and 11.7% anhydrous citric acid (which react in water to form magnesium citrate in the growth medium), 43.1% whey solvents, 31.1% dibasic ammonium phosphate (compound B) and 5.8% yeast extract to produce 1,361 kg (3,000 pounds) and blended together in a tumbler bin. The product was used to grow bacteria as before (34 kg/379 l (75 pounds/100 gallons of water)), wherein magnesium ammonium phosphate is formed *in situ*. The bacteria produced were excellent as before and the intial pH and final pH were about the same as before. Extensive field testing of the product resulted in a cement-like cake on the bottom of the starter tank for some trials and not for other trials (like Example 2). Also, analysis of citrate concentration of citric acid showed high variations of available citrate on the order of four-fold as observed with trisodium citrate and as shown in Example 2. Thus, the alternate method for *in situ* generation of neutralizing agent which uses citric acid as in Example 1 rather than trisodium citrate like Example 2, provided a media very similar chemically in compositions to the Examples 1 and 2 but the characteristics were different from Example 1 and simlar to Example 2.

If uniform quality citric acid or sodium citrate could be obtained, the results would be more comparable to Example 1. However, magnesium hyroxide may contribute to the cement-like by product.

Example 4

Commercial ultra-high temperature short time pasteurization is sometimes accomplished by pumping an aqueous bacterial growth medium through a tightly packed heated plate pasteurizer at temperatures above 93.5°C (200°F). With some media in EP—A—0 022 341, and the media described in Examples 1 to 3 of this application, there is often a build-up of insoluble media ingredients on the pasteurizer plate which results in plugging. However, a medium which consists of two separately pasteurized parts followed by *in situ* generation of the neutralizing agent works well. For each 379 l (100 gallons) of starter to be prepared, about 6.5 kg (14 pounds) of a mixture of magnesium hydroxide (39.9%) and citric acid monohydrate (60.1%) can be added to 155 l (41 gallons) of water to form magnesium citrate (Compound A) in solution and then pasteurized with a ultra-high temperature short time unit without problems since the solids are mostly dissolved. Then 11 kg (24 pounds) of the mixture of dibasic ammonium phosphate (67.2%), yeast extract (16.6%), and monobasic ammonium phosphate (16.2%) can be added to 212 l (56 gallons) of suitable aqueous medium such as water containing whey solids or skimmed milk which can be pasteurized with the ultra high temperature short time pasteurizer. The liquid is then mixed into the stirring tank containing the pasteurized magnesium citrate. After combination, the insoluble neutralizing agent magnesium ammonium phosphate is generated *in situ* and the media is excellent for growing lactic acid producing bacteria.

Example 5

A growth medium, No. 1 below, was prepared by mixing 5.5 ml (7.3 grams) of phosphoric acid and 200 ml of tap water followed by 5.2 grams of magnesium hydroxide powder with stirring for 10 minutes. Heat is given off and the temperature rises to 57°C. This forms a magnesium phosphate which has limited solubility in water. Then 500 ml of tap water was added followed by 10.5 grams of dibasic ammonium phosphate, 10.5 grams of trisodium citrate dihydrate, 3.5 grams yeast extract and 24.5 grams whey powder. This medium allowed a chemical reaction to occur *in situ* to generate a magnesium phosphate and magnesium ammonium phosphate complex by using readily available and inexpensive starting materials rather than using an expensive and limited availability material, e.g. magnesium phosphate and magnesium ammonium phosphate. For comparison, another growth medium, No. 2 below, as in EP—A—0 022 341, Example 10 was also prepared by blending together the dry powder 10.5 grams magnesium phosphate tribasic, 10.5 grams disbasic ammonium phosphate, 10.5 grams trisodium citrate dihydrate, 3.5 grams yeast extract and 24.5 grams whey powder, followed by addition to 700 ml of water. Both preparations were continuously stirred, heated to 85°C within 20 minutes, maintained at 85°C for 45 minutes and cooled to 24°C and inoculated in successive trials with commercially available streptococcal lactic acid producing bacteria. Also evaluated as a control was a growth medium (No. 3 below) containing 11% non-fat dry milk solids and 700 ml of tap water, heated and cooled and inoculated as stated above. The pH time course of media were as shown in Table 1:

TABLE 1
Recorded pH of the media

| Elapsed time in hours | Culture DPL102 | | | Culture DPL104 | | | Culture HAN72 | |
|---|---|---|---|---|---|---|---|---|
| | 1 | 2 | 3 | 1 | 2 | 3 | 1 | 3 |
| 0 | 7.00 | 6.92 | 6.46 | 6.84 | 6.44 | 6.41 | 7.01 | 6.46 |
| 13 | 5.12 | 5.06 | 4.59 | 5.50 | 5.17 | 4.99 | 5.28 | 4.80 |
| 14 | 5.11 | 5.10 | 4.56 | 5.34 | 5.08 | 4.78 | 5.28 | 4.82 |
| 15 | 5.11 | 4.98 | 4.57 | 5.33 | 5.64 | 4.70 | 5.28 | 4.81 |
| 16 | 5.09 | 4.95 | 4.57 | 5.32 | 5.04 | 4.66 | 5.28 | 4.82 |

The medium 1, which contains an *in situ* generated neutralizing agent and is much less expensive to prepare than medium 2, is equally effective in maintaining the growth medium pH in a range which is known to be conducive to growth of healthy active cells while the pH falls too low in the non-fat milk control medium (medium 3).

Activity tests of bacteria grown in the above media 1 and 3 were also conducted by inoculating culture HAN 72 (1%) into non-fat dry milk (11% by weight solids) and measuring the pH for four hours and six hours post inoculation for freshly grown cells and for cells maintained in the refrigerator for eight days. The four and six hour pH values were: 5.69 and 4.98 (cells from medium 1) vs. 6.07 and 5.50 (cells from medium 3) for fresh cells and 5.78 and 5.02 vs. 6.12 and 5.47 for eight day old cells, respectively. It is clear that cells grown in medium 1 are more active initially and after storage than cells grown in medium 3. Better acid control was achieved with medium 1 as compared to medium 2.

Either the mono or dibasic ammonium phosphate can serve as a source of ammonium phosphate anion. The diammonium phosphate is believed to react first.

As can be seen, the *in situ* method of generating the neutralizing agent is very effective.

**Claims**

1. In the method for growing acid producing bacteria in an aqueous growth medium including bacterial nutrients, the improvement which comprises: reacting in the aqueous growth medium an alkaline earth metal cation in a compound A with an anion in a compound B which reacts with the alkaline earth metal cation to form an essentially water insoluble salt, base or mixture thereof including the alkaline earth metal cation and the anion, which essentially water insoluble salt base or mixture thereof is acid neutralizing in the medium, wherein the resulting medium includes an amount of the insoluble salt, base or mixture thereof in the medium sufficient to neutralize at least some of the acid generated by the acid producing bacteria and wherein the resulting medium is non-toxic to the bacteria.

2. The method of Claim 1 wherein the alkali metal cation is selected from calcium and magnesium.

3. The method of Claim 1 wherein compound A contains a magnesium cation and compound B includes a phosphate anion which are admixed and then added to the aqueous growth medium as a powder.

4. The method of Claim 1 wherein compound A is magnesium citrate, compound B is an ammonium phosphate, and the insoluble salt formed in the aqueous growth medium is essentially magnesium ammonium phosphate.

5. The method of Claim 4 wherein the magnesium citrate is provided by reacting magnesium hydroxide with citric acid in an aqueous solution to an essentially neutral pH and spray drying the magnesium citrate to a powder.

6. An aqueous growth medium containing an essentially water insoluble salt, base or mixture thereof formed according to the method of Claim 1.

7. A composition which is adapted to be mixed with water to form an aqueous growth medium for acid-producing bacteria, which composition comprises a water soluble, alkaline earth metal cation in a compound A admixed with an anion in a compound B in an amount sufficient to react in solution when mixed with water to form an essentially water insoluble salt, base or mixture thereof, including the alkaline earth metal cation and the anion, wherein the composition is adapted to provide an amount of the insoluble salt, base or mixture thereof in the aqueous medium sufficient to neutralize at least some of the acid generated by the acid producing bacteria and wherein the resulting medium is non-toxic to the bacteria.

8. The composition of Claim 7 wherein the alkaine earth metal cation is selected from calcium and magnesium.

9. The composition of Claim 7 or 8, wherein the powdered medium contains between about 5 and 30 weight percent each of compounds A and B.

10. A composition according to any one of Claims 7 to 9, further comprising bacterial nutrients.

11. A composition according to any one of Claims 7 to 10, wherein the anion is an hydroxide carbonate or phosphate ion.

12. A composition according to any one of Claims 7 to 11, wherein compound A contains citrate.

13. A composition according to any one of Claims 7 to 12, wherein compound B contains ammonium ions or alkali metal cations.

14. A composition according to any one of Claims 7 to 13, wherein the essentially water soluble salt is magnesium ammonium phosphate.

15. The composition of Claim 14, wherein compound A is magnesium citrate, compound B is an ammonium phosphate, and the insoluble salt is essentially magnesium ammonium phosphate.

16. A composition according to any one of Claims 7 to 15, which is a dry powder.

17. A composition according to any one of Claims 7 to 16, wherein the aqueous growth medium produced by mixing the composition with water has an initial pH between about 4 and 8.5.

18. An aqueous growth medium whenever produced by mixing a composition according to any one of Claims 7 to 17 with water.

**Patentansprüche**

1. Im Verfahren zum Züchten von säureproduzierenden Bakterien in einem wässrigen Wachstumsmedium, das Bakteriennährstoffe enthält, die Verbesserung, welche umfaßt: im wässrigen Wachstumsmedium Umsetzen eines Erdalkalimetallkations in einer Verbindung A mit einem Anion in einer Verbindung B, das mit dem Erdalkalimetallkation unter Bildung eines im wesentlichen wasserunlöslichen Salzes, einer im wesentlichen wasserunlöslichen Base oder einer im wesentlichen wasserunlöslichen Mischung derselben, welches (welche) das Erdalkalimetallkation und das Anion enthält, reagiert, wobei das im wesentlichen wasserunlösliche Salz, die im wesentlichen wasserunlöslichen Base oder im wesentlichen wasserunlösliche Mischung derselben im Medium säureneutralisierend ist, wobei das resultierende Medium eine Menge des unlöslichen Salzes, der unlöslichen Base oder unlöslichen Mischung derselben im Medium enthält, die hinreichend ist, wenigstens einen Teil der Säure, die von den säureproduzierenden Bakterien erzeugt wird, zu neutralisieren, und wobei das resultierende Medium für die Bakterien ungiftig ist.

2. Verfahren des Anspruchs 1, wobei das Alkalimetallkation aus Calcium und Magnesium ausgewählt ist.

3. Verfahren des Anspruchs 1, wobei die Verbindung A ein Magnesiumkation und die Verbindung B ein Phosphatanion enthält, die vermischt und dann dem wässrigen Wachstumsmedium als Pulver zugefügt werden.

4. Verfahren des Anspruchs 1, wobei die Verbindung A ein Magnesiumzitrat, die Verbindung B ein Ammoniumphosphat und das unlösliche Salz, das im wässrigen Wachstumsmedium gebildet ist, im wesentlichen Magnesiumammoniumphosphat ist.

5. Verfahren des Anspruchs 4, wobei das Magnesiumzitrat dadurch geliefert wird, daß Magnesiumhydroxid mit Zitronensäure in einer wässrigen Lösung bis zu einem im wesentlichen neutralen pH umgesetzt und das Magnesiumzitrat zu einem Pulver sprühgetrocknet wird.

6. Wässriges Wachstumsmedium, das ein im wesentlichen wasserunlösliches Salz, eine im wesentlichen wasserunlösliche Base oder eine im wesentlichen wasserunlösliche Mischung derselben, hergestellt nach dem Verfahren des Anspruchs 1, enthält.

7. Zusammensetzung, welche angepaßt ist, um mit Wasser gemischt zu werden, um ein wässriges Wachstumsmedium für säureproduzierende Bakterien zu bilden, wobei diese Zusammensetzung ein wasserlösliches Erdalkalimetallkation in einer Verbindung A gemischt mit einem Anion in einer Verbindung B in einer Menge aufweist, die hinreicht, um in Lösung zu reagieren, wenn sie mit Wasser gemischt wird, um ein im wesentlichen wasserunlösliches Salz, eine im wesentlichen wasserunlösliche Base oder ein im wesentlichen wasserunlösliche Mischung derselben, welches (welche) das Erdalkalimetallkation und Anion enthält, zu bilden, wobei die Zusammensetzung angepaßt ist, um eine Menge an unlöslichem Salz, an unlöslicher Base oder an unlöslicher Mischung derselben im wässrigen Medium zu liefern, die hinreicht, wenigstens einen Teil de Säure, die von den säureproduzierenden Bakterien erzeugt wird, zu neutralisieren, und wobei das resultierende Medium für die Bakterien ungiftig ist.

8. Zusammensetzung des Anspruchs 7, wobei das Erdalkalimetallkation aus Calcium und Magnesium ausgewählt ist.

9. Zusammensetzung des Anspruchs 7 oder 8, wobei das pulverisierte Medium zwischen etwa 5 und 30 Gew.-% jeder der Verbindungen A und B enthält.

10. Zusammensetzung nach einem der Ansprüche 7 bis 9, die weiterhin Bakteriennährstoffe aufweist.

11. Zusammensetzung nach einem der Ansprüche 7 bis 10, wobei das Anion ein Hydroxid-, Carbonat- oder Phosphation ist.

12. Zusammensetzung nach einem der Ansprüche 7 bis 11, wobei die Verbindung A Zitrat enthält.

13. Zusammensetzung nach einem der Ansprüche 7 bis 12, wobei die Verbindung B Ammoniumionen oder Alkalimetallkationen enthält.

14. Zusammensetzung nach einem der Ansprüche 7 bis 13, wobei das im wesentlichen wasserunlösliche Salz Magnesiumammoniumphosphat ist.

15. Zusammensetzung des Anspruchs 14, wobei die Verbindung A Magnesiumzitrat, die Verbindung B ein Ammoniumphophat und das unlösliche Salz im wesentlichen Magnesiumammoniumphosphat ist.

16. Zusammensetzung nach einem der Ansprüche 7 bis 15, welche ein trockenes Pulver ist.

17. Zusammensetzung nach einem der Ansprüche 7 bis 16, wobei das wässrige Wachstumsmedium, das durch Mischen der Zusammensetzung mit Wasser hergestellt wird, einen anfänglichen pH zwischen etwa 4 und 8,5 besitzt.

18. Wässriges Wachstumsmedium, wann immer dieses durch Mischen einer Zusammensetzung nach einem der Ansprüche 7 bis 17 mit Wasser hergestellt wird.

**Revendications**

1. Procédé pour la culture de bactéries produisant de l'acide dans un milieu aqueux de culture comprenant des éléments nutritifs bactériens, auquel on apporte l'amélioration qui consiste en ce que l'on fait réagir dans le milieu aqueux de culture un cation métal alcalinoterreux d'un composé A avec un anion d'un composé B qui réagit avec le cation métal alcalino-terreux pour former un sel, une base ou un mélange de, sel et base, essentiellement insoluble dans l'eau et comprenant le cation métal alcalino-terreux et l'anion, ce sel, cette base ou ce mélange de sel et base, essentiellement insoluble dans l'eau, étant susceptible de neutraliser l'acide dans le milieu, et dans laquelle le milieu obtenu comprend une quantité du sel, de la base ou de mélange de sel et base, insoluble, suffisante pour neutraliser au moins une partie de l'acide généré par les bactéries produisant de l'acide, et dans laquelle le milieu obtenu est non-toxique pour les bactéries.

2. Procédé conforme à la revendication 1, dans lequel le cation métal alcalino-terreux est choisi parmi le calcium et le magnésium.

3. Procédé conforme à la revendication 1, dans lequel le composé A contient un cation magnésium et le composé B contient un anion phosphate qui sont mélangés puis ajoutés au milieu aqueux de culture à l'état de poudre.

4. Procédé conforme à la revendication 1, dans lequel le composé A est du citrate de magnésium, le composé B est un phosphate d'ammonium, et le sel insoluble formé dans le milieu aqueux de culture est essentiellement du phosphate double de magnésium et d'ammonium.

5. Procédé conforme à la revendication 4, dans lequel le citrate de magnésium est fourni par réaction d'hydroxyde de magnésium avec de l'acide citrique dans une solution aqueuse à un pH essentiellement neutre, et obtention du citrate de magnésium à l'état de poudre par séchage par pulvérisation.

6. Milieu aqueux de culture contenant un sel, une base ou un mélange de sel et base, essentiellement insoluble dans l'eau, obtenu selon le procédé de la revendication 1.

7. Composition destinée à être mélangée à de l'eau pour former un milieu aqueux de culture pour bactéries produisant de l'acide, cette composition comprenant un cation métal alcalino-terreux soluble dans l'eau contenu dans une composé A mélangé à un anion contenu dans un composé B en quantité suffisante pour réagir en solution quand ils sont mélangés à de l'eau de manière à former un sel, une base ou un mélange de sel et base, essentiellement insoluble dans l'eau, contenant le cation métal alcalino-terreux et l'anion, dans laquelle la composition est adaptée à fournir dans le milieu aqueux une quantité de sel, de base ou de mélange de sel et base, insoluble, suffisante pour neutraliser au moins une partie de l'acide généré par les bactéries produisant de l'acide, et dans laquelle le milieu obtenu est non-toxique pour les bactéries.

8. Composition conforme à la revendication 7, dans laquelle le cation métal alcalino-terreux est choisi parmi le calcium et le magnésium.

9. Composition conforme à la revendication 7 ou 8, dans laquelle le milieu en poudre contient entre 5 et 30 pour cent environ en poids de chacun des composés A et B.

10. Composition conforme à l'une quelconque des revendications 7 à 9, qui comprend de plus de éléments nutritifs bactériens.

11. Composition conforme à l'une quelconque des revendications 7 à 10, dans laquelle l'anion est un ion hydroxyde, carbonate ou phosphate.

12. Composition conforme à l'une quelconque des revendications 7 à 11, dans laquelle le composé A contient du citrate.

13. Composition conformé à l'une quelconque des revendications 7 à 12, dans laquelle le composé B contient des ions ammonium ou des cations métaux alcalins.

14. Composition conforme à l'une quelconque des revendications 7 à 13, dans laquelle le sel essentiellement insoluble dans l'eau est du phosphate double de magnésium et d'ammonium.

15. Composition conforme à la revendication 14, dans laquelle le composé A et du citrate de magnésium, le composé B est un phosphate d'ammonium, et le sel insoluble est essentiellement du phosphate double de magnésium et d'ammonium.

16. Composition conforme à l'une quelconque des revendications 7 à 15, qui est sous forme d'une poudre sèche.

17. Composition conforme à l'une quelconque des revendications 7 à 16, dans laquelle le milieu aqueux de culture produit en mélangeant la composition avec de l'eau à un pH initial entre 4 et 8,5 environ.

18. Milieu aqueux de culture produit en mélangeant une composition conforme à l'une quelconque des revendications 7 à 17 avec de l'eau.